(19) Europäisches Patentamt
European Patent Office
Office européen des brevets

(11) Publication number: **0 454 321 A2**

(12) # EUROPEAN PATENT APPLICATION

(21) Application number: **91303192.8**

(51) Int. Cl.[5]: **C07H 15/04,** // C11D1/12

(22) Date of filing: **11.04.91**

(30) Priority: **27.04.90 JP 113773/90**
**21.11.90 JP 320056/90**

(43) Date of publication of application:
**30.10.91 Bulletin 91/44**

(84) Designated Contracting States:
**AT CH DE ES FR GB LI NL**

(71) Applicant: **KAO CORPORATION**
**14-10, Nihonbashi Kayabacho 1-chome**
**Chuo-ku Tokyo (JP)**

(72) Inventor: **Mizushima, Hiromoto**
**44, Tosacho 1-chome**
**Wakayama-shi, Wakayama (JP)**
Inventor: **Yamamuro, Akira**
**Seiwaryo, 1130, Nishihama**
**Wakayama-shi, Wakayama (JP)**
Inventor: **Yokota, Yukinaga**
**208-10, Tottorinaka, Hannancho**
**Sennan-gun, Osaka (JP)**

(74) Representative: **Bannerman, David Gardner et al**
**Withers & Rogers 4 Dyer's Buildings Holborn**
**London, EC1N 2JT (GB)**

(54) **Glycoside ester of sulfosuccinic acid and process for producing the same.**

(57) A glycoside ester of sulfosuccinic acid useful as a surfactant is defined by the formula (I) in which Gn is a sugar residue, A is alkyl to which an alkylene oxide residue has been added, R' is alkylene, M is hydrogen, metal or ammonium and B is hydrogen or

```
-CO-CH2CH-CO2M,
        |
        SO3M
```

provided that at least one of the B residue is

```
-CO-CH2CH-CO2M.

        SO3M
```

The glycoside ester may be produced by reacting a glycoside compound with maleic anhydride and then an alkali metal sulfate.

A(Gn)[(R'O)xB]y (I)

EP 0 454 321 A2

A half ester of sulfosuccinic acid (herein meaning an ester of sulfosuccinic acid formed by esterifying only one of the two carboxyl groups of the acid which is bound to the carbon atom to which the sulfo group is bound) is a low-irritating anionic surfactant and therefore used as shampoos and a detergent base for bubble baths in Europe and America. It is known that the half esters of sulfosuccinic acid are not only substantially non-irritating per se but also can remarkably reduce the irritation of the skin and the mucous membrane of the eye caused by other surfactants, when used together with such other surfactants (see Japanese Patent Laid-Open No.38395/1981), and their use has been proposed as a component for detergent compositions such as hair shampoos, body shampoos or hand washing soaps which are low irritating, excellent in rinsability and capable of imparting softness (Japanese Patent Laid-Open No. 65798/1982). However, these half esters have the defect that they usually have relatively-poor lathering properties.

On the other hand, alkylglycosides which are surfactants based on sugar derivatives are low irritating and have such excellent properties that they can form stable foams per se and act as foam stabilizers for anionic surfactants, though they themselves are nonionic surfactants. Recently they have attracted attention from the viewpoint of environmental protection, because they are available through utilization of biomass materials which are independent of fossil fuels and have good biodegradability.

However, the alkylglycosides display poor water solubility. Therefore various investigations are known to have been carried out with a view to increasing the solubility of alkylglycosides by chemically modifying them to increase their suitability for use as surfactants. For example, U.S. Patent Nos. 3 640 998 and 3 653 095 and EP-A 277 944 disclose modification of alkylglycosides by an addition reaction with alkylene oxides. U.S. Patent No. 4,663,444 discloses the synthesis of alkyl-α-glycoside 6-0-mono-(long-chain alkyl) ethers by the reaction of an alkyl-α-glycoside with a long-chain alkyl methanesulfonate. In addition, EP-A 326 673 discloses a process for producing alkylglucuronic acids by oxidizing an alkylglycoside in an aqueous solvent and in the presence of a platinum catalyst. However, the properties of the resultant alkylglycoside derivatives are either not superior or are inferior to those of the starting alkylglycosides, or their synthesis on an industrial scale is difficult.

After intensive investigations, we have found that glycoside esters of sulfosuccinic acid which are derived from alkylglycosides are free from problems that detract from their use as surfactants, i.e. the poor lathering properties displayed by known half esters of sulfosuccinic acid and the low water solubility of the alkylglycosides. In addition, they are excellent surfactants which are environmentally unobjectionable and are harmless towards the human body, since they are highly biodegradable and only slightly irritating. The present invention has been completed on the basis of these findings.

Thus the present invention provides glycoside esters of sulfosuccinic acid of the following general formula (I):

$$A(Gn)[(R'0)xB]y \qquad (I)$$

wherein:

Gn represents a sugar residue remaining after removing hydrogen atoms from all of the non-glycosinic hydroxyl groups and glycosidic hydroxyl groups of a reducing sugar having 5 or 6 carbon atoms or a condensate thereof in which n represents the degree of the condensation which has an average value from 1 to 10;

A represents an $R^2(OR^3)_z$ group bound to the sugar residue Gn through an O-glycoside bond in which $R^2$ represents a straight chain or branched alkyl, alkenyl or alkylphenyl group having 6 to 22 carbon atoms, $R^3$ represents an alkylene group having 2 to 4 carbon atoms and z represents a number having an average value of 0 to 20;

$R^1$ represents an alkylene group having 2 to 4 carbon atoms, one end of which forms an ether bond with an oxygen atom of a non-glycosidic hydroxyl group of the saccharide residue Gn and the other end of which forms an ester bond with a sulfosuccinoyl group;

x represents the ratio of the total molar number of alkylene oxide groups added to the non-glycosidic hydroxyl groups of the reducing sugar having 5 or 6 carbon atoms or a condensate thereof to y, and has a value from 0 to 10;

y represents the number of non-glycosidic hydroxyl groups in the reducing sugar having 5 or 6 carbon atoms or a condensate thereof; and

B is hydrogen or

$$-CO-CH_2\underset{\displaystyle SO_3M}{\underset{|}{C}H}-CO_2M,$$

provided that at least one B residue is

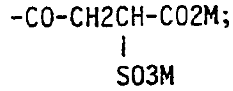

and M represents residues that may be the same or different from each other and each is a hydrogen atom, an alkli metal, an alkaline earth metal, ammonium, mono-di- or trialkanolammonium having 2 or 3 carbon atoms, an alkyl-substituted ammonium having 1 to 5 carbon atoms, or a basic amino acid group. The invention also provides a process for the production of these glycoside esters.

Examples of Gn as defined above are as follows:

Gn :

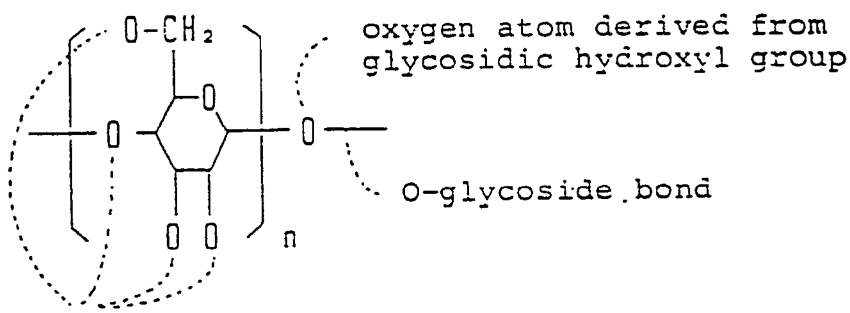


oxygen atom derived from non-glycosidic hydroxyl group

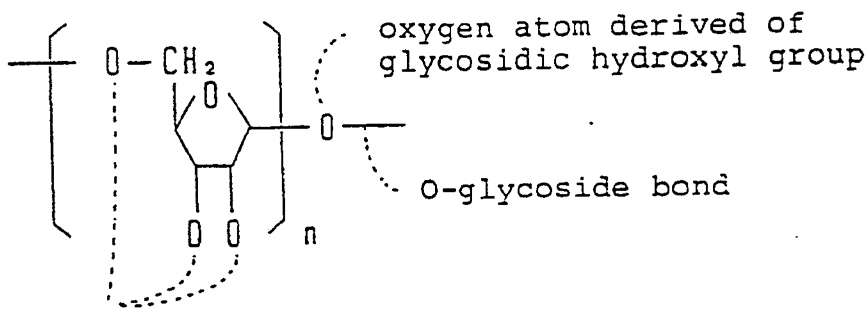


oxygen atom derived from non-glycosidic hydroxyl group

wherein n represents the degree of bonding of the sugar and has an average value from 1 to 10.

Processes for producing half esters of sulfosuccinic acid have long been known. U.S. Patent No. 2,628,091 discloses a process wherein maleic anhydride is reacted with an aliphatic alcohol and the reaction product is sulfated with sodium sulfite. Half esters of sulfosuccinic acid with various alcohols are known, including those with ethylene oxide adducts of alcohols having 12 to 18 carbon atoms (DE 3627296), those with polyethylene oxide or polypropylene oxide (DE 311601), and those with perfluoroalkyl alcohols or their ethylene oxide adducts (U.S. Patent 4 167 639).

The glycoside esters of sulfosuccinic acid of the present invention are half esters of sulfosuccinic acid having a glycoside group (Gn) and usable as new surfactants having excellent properties. The glycoside esters of sulfosuccinic acid of the present invention may be produced by reacting a glycoside compound or an alkylene oxide adduct thereof of the following general formula (II) or mixture thereof with maleic anhydride to form a half ester of maleic acid of the following general formula (III), neutralizing this half ester, if necessary, with an alkaline substance and sulfonating it with an alkali metal sulfite and/or an acidic alkali metal sulfite containing an alkali metal hydroxide:

$$A(Gn)[(R'O)xH]y \qquad (II)$$

wherein A, Gn, x and y are as defined above, and $R^1$ represents an alkylene group having 2 to 4 carbon atoms, one end of which forms an ether bond with an oxygen atom of a non-glycosidic hydroxyl group of the sugar residue Gn and the other end of which is bound to a hydroxyl group, and

$$A(Gn)[(R'O)xE]y \qquad (III)$$

wherein A, Gn, x and y are as defined above, and

$R^1$ represents an alkylene group having 2 to 4 carbon atoms, one end of which forms an ether bond with an oxygen atom of a non-glycosidic hydroxyl group of the sugar residue Gn and the other end of which is bound to the ester group;

E is hydrogen or -CO-CH=CH-SO3M, provided that at least one of the E residues is -CO-CH=CH-SO3M; and the M residues which may be the same or different from each other,

each represent a hydrogen atom, an alkali metal, an alkaline earth metal, ammonium, mono-, di- or trialkanolammonium having 2 or 3 carbon atoms, an alkyl-substituted ammonium having 1 to 5 carbon atoms, or a basic amino acid group.

Glycoside compound (II) used as starting material for glycoside ester of sulfosuccinic acid (I) :

The glycoside compounds (II) used in the present invention are synthesized by a known process (see U.S. patent 3 598 865, U.S. patent 3 839 318, EP-A 092 355, EP-A 77167 and EP-A 92355 Patent Laid-Open Nos. 139397/1984 and 189195/1983). They are obtained by a process in which a sugar is directly reacted with a higher alcohol in the presence of an acid catalyst or a process in which a sugar is first reacted with a lower alcohol such as methanol, ethanol, propanol or butanol and the resulting product is reacted with a higher alcohol. The glycoside compound thus obtained can be used in the form of an adduct with an alkylene oxide having 2 to 4 carbon atoms.

The sugars which may be used for the synthesis of the glycoside compounds (II) include monosaccharides, oligosaccharides and polysaccharides. Examples of suitable monosaccharides include aldoses such as allose, altrose, glucose, mannose, gulose, idose, galactose, talose, ribose, arabinose, xylose and lyxose; and ketoses such as fructose. Examples of suitable oligosaccharides include maltose, lactose sucrose and maltotriose. Examples of suitable polysaccharides include hemicellulose, inulin, dextrin, dextran, xylan, starch and hydrolyzed starch.

The higher alcohols used for the synthesis of the glycoside compounds (II) are straight chain or branched alcohols having 6 to 22 carbon atoms, such as hexanol, heptanol, octanol, nonanol, decanol, dodecanol, tridecanol, tetradecanol, pentadecanol, hexadecanol, heptadecanol, octadecanol, methylpentanol, methylhexanol, methylheptanol, methyloctanol, methyldecanol, methylundecanol, methyltridecanol, methylheptadecanol, ethylhexanol, ethyloctanol, ethyldecanol, ethyldodecanol, 2-heptanol, 2-nonanol, 2-undecanol, 2-tridecanol, 2-pentadecanol, 2-heptadecanol, 2-butyloctanol, 2-hexyloctanol, 2-octyloctanol, 2-hexyldecanol and 2-octyldecanol. Examples of the alkenols include hexenol, heptenol, octenol, nonenol, decenol, undecenol, dodecenol, tridecenol, tetradecenol, pentadecenol, hexadecenol, heptadecenol and octadecenol. Examples of the alkylphenols include octylphenol and nonylphenol. Further the $C_2$ to $C_4$ alkylene oxide adducts of the above-described higher alcohols or alkylphenols are also usable.

The glycoside compounds (II) thus obtained are esterified and then sulfonated by the following processes to form the glycoside esters of sulfosuccinic acid (I) of the present invention:

glycoside ester of sulfosuccinic acid (I):

For the synthesis of the half esters of sulfosuccinic acid, a process is known which comprises reacting maleic anhydride with an aliphatic alcohol to form a half ester and then sulfonating the half ester with sodium sulfite in an aqueous solution (see U.S. Patent No. 2,628,091 and DE 3,627,296).

The glycoside ester of sulfosuccinic acid of the present invention can be synthesized by reacting a starting glycoside compound (II) with maleic anhydride in a non-aqueous, non-alcoholic solvent to form a half ester and then sulfonating the half ester with sodium sulfite or the like in an aqueous solvent in the same manner as that of the above-described known process. It is important in the present invention that only one of the two carboxyl groups of starting maleic anhydride be esterified with a non-glycosidic hydroxyl group of the starting glycoside compound or with a hydroxyl group of an alkylene oxide adduct thereof. Hereinafter, an ester formed by esterifying only one of the two carboxyl groups of a cyclic acid anhydride will be referred to as "half ester" and an ester formed by esterifying both of the two carboxyl groups will be referred to as "diester".

After intensive investigation of processes for the synthesis of the glycoside esters of sulfosuccinic acid, the inventors hare found it possible to carry out the reaction without using any solvent or preferably to selectively obtain the intended half ester by conducting the esterification reaction in a non-aqueous, non-alcoholic solvent. The glycoside ester of sulfosuccinic acid (I) can successively be formed in a high production yield by subsequently sulfonating the half ester with sodium sulfite or the like in water as the solvent. The process of the present invention has been completed on the basis of these findings.

. In particular, the sulfosuccinic ester (I) of the present invention can be efficiently obtained by dissolving the starting glycoside compound (II) of the present invention in a non-aqueous, non-alcoholic solvent, then adding maleic anhydride to the glycoside solution, stirring the mixture by heating under nonaqueous conditions to obtain the half ester of maleic anhydride, and then stirring it by heating in an aqueous sodium sulfite solution.

The scheme of these reactions is illustrated as follows:

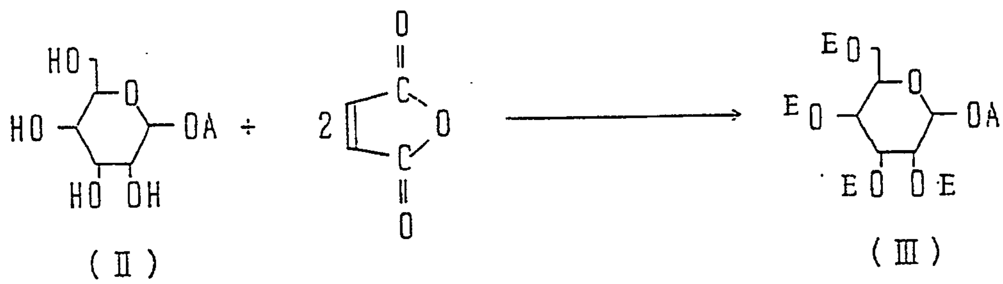

(II) (III)

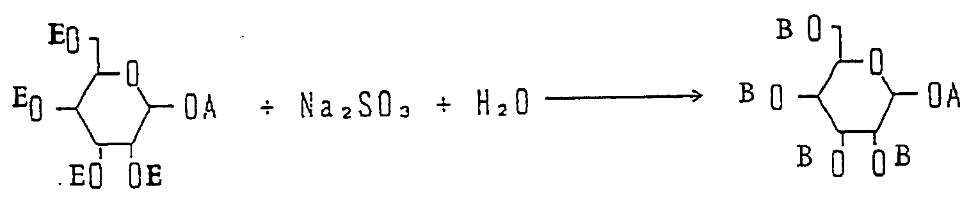

(III) (I)

wherein groups E and B are as defined above, with the proviso that two of the groups E each represent

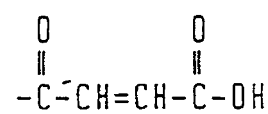

group and two other groups E each represent a hydrogen atom, and two of the groups B each represent

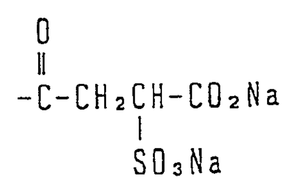

group and two other groups B each represent a hydrogen atom.

In the esterification step in the reactions of the present invention, a non-aqueous, non-lower-alcoholic organic solvent in which the starting glycoside compound (II) is soluble is used. The organic solvents usable herein include DMF, DME, DMSO, dioxane, THF, toluene and ethyl acetate, among which DMF and toluene are desirable. Although the esterification reaction can be conducted without using any solvent, it is preferred to conduct it in a solvent, since when the reaction is conducted in the absence of any solvent, a quite high reaction temperature is necessary in order to lower the viscosity of the mixture of the starting glycoside compound (II) and maleic anhydride and, as a result, side reactions such as a diester forming reaction easily occur. The amount of starting maleic anhydride can be selected depending on the degree of esterification of the intended glycoside ester of sulfosuccinic acid (I).

The reaction temperature is 0 to 150°C, preferably 20 to 90°C. When the temperature is above 90°C, side reactions such as the diester-forming reaction are accelerated unfavorably. When the temperature is too low on the contrary, a long time is required in the esterification unfavorably. The esterification time of 1 to 20 h is sufficient, though it varies depending on the temperature.

Thereafter, the solvent used in the esterification reaction is distilled off by heating under reduced pressure. The glycoside half ester of maleic anhydride thus obtained is of carboxylic acid type, which can be neutralized, if necessary, with an alkaline substance to form a neutralized salt to be subjected to the subsequent sulfonation step. The alkaline substance is dissolved in water to form a 1 to 40% aqueous solution when it is solid, and

then added to the carboxylic acid in an equivalent amount and the mixture is stirred at a temperature of 70°C or below for about 1 h to obtain the neutral salt (neutralization step). Examples of the alkaline substances include inorganic alkalis such as sodium hydroxide, potassium hydroxide, lithium hydroxide, barium hydroxide, sodium hydrogencarbonate, sodium carbonate and potassium carbonate, as well as monoethanolamine, diethanolamine, triethanolamine and basic amino acids.

The maleic half ester of glycoside or its neutral salt thus obtained is reacted in an aqueous solution of an alkali metal sulfite or acidic alkali metal sulfite at 50 to 100°C, preferably 60 to 90°C, for 2 to 5 h to easily conduct the sulfonation (sulfonation step).

Water contained in the resultant mixture containing the glycoside ester of sulfosuccinic acid (I) is distilled off by heating under reduced pressure (dehydration step) and inorganic and organic salts still remaining therein can be removed by electrodialysis, adsorption with an ion exchange resin or adsorbent, or solvent refining (desalting step). The above-described neutralization step, dehydration step and desalting step can be omitted depending on the use of the product.

The glycoside esters of sulfosuccinic acid (I) obtained as described above are new anionic surfactants having ester and glycoside groups having a good biodegradability. They have mild effects on the skin and hair and a high safety to the human beings, other living things and the environment. They have excellent interfacial properties such as lathering properties, resistance to hard water and solubility in water and can be widely used in various fields as, for example, detergents.

[Examples]

The following non-limiting Examples will further illustrate the present invention:

Example 1

a) 4890 g (26.3 mol) of lauryl alcohol, 789 g (4.38 mol) of anhydrous glucose and 11.7 g (0.06 mol) of p-toluenesulfonic acid monohydrate were stirred under heating in a 10-ℓ flask. After elevating the temperature to 100°C, the pressure in the flask was adjusted to 40 mmHg and the dehydration reaction was initiated. 0.1 $Nm^3/h$ of nitrogen was introduced into the liquid reaction mixture to efficiently remove water formed by the reaction. 7.5 h after initiation of the reaction, the consumption of glucose was macroscopically confirmed. The pressure was allowed to rise to an atmospheric pressure, the reaction mixture was cooled and an aqueous NaOH solution was added thereto to neutralize it. Polysaccharides formed as by products were filtered off and the filtrate was distilled at 180°C under 0.3 mmHg to obtain 1,400 g of laurylglucoside. The laurylglucoside thus obtained had an average degree of sugar condensation of 1.2.

b) 50 g (0.131 mol) of the laurylglucoside obtained as described above was dissolved in 50 mℓ of DMF. 12.9 g (0.131 mol) of finely pulverized maleic anhydride was added thereto and the mixture was stirred at 60°C for 2 h. 200 mℓ of an aqueous solution of 16.5 g (0.131 mol) of sodium sulfite was added to the reaction mixture and the mixture was stirred at 80°C for about 3 h. Water and DMF were distilled off from the mixture by heating in an evaporator under reduced pressure. The remaining solid was washed with about 500 mℓ of ethanol to obtain 70 g of a white powder (yield: 85.6% based on laurylglucoside). The IR data given below suggested the formation of laurylglucoside ester of sulfosuccinic acid.

IR ($cm^{-1}$, KBr tablet method):

3460 (OH)
2926 and 2860 (C-H)
1731 (ester carbonyl)
1614 and 1410 ($CO_2Na$)
1230 and 1047 ($SO_3Na$)

Mass spectrum (FAB ionization method):

Molecular ion peaks (+H type and +Na type* 1) corresponding to disodium salt of laurylmonoglucoside half

(Note)* In the FAB ionization method, the molecular ion peaks of polar substances often appear as +Na-type cluster.

ester of monosulfosuccinic acid and disodium salt of lauryldiglucoside half ester of monosulfosuccinic acid were detected:

disodium salt of laurylmonoglucoside half ester of monosulfosuccinic acid:

$$(C_{22}H_{38}O_{12}SNa_2 = 572): \quad 573\ (M+H)^+,\ 595\ (M+Na)^+,\ 1145\ (2M+H)^+,\ 1167\ (2M+Na)^+$$

disodium salt of lauryldiglucoside half ester of monosulfosuccinic acid:

$$(C_{28}H_{48}O_{17}SNa_2 = 734): \quad 735\ (M+H)^+,\ 757\ (M+Na)^+$$

Example 2

300 g (0.772 mol) of laurylglucoside having a degree of sugar condensation of 1.25 was dissolved in 300 g of toluene. 71.9 g (0.733 mol) of maleic anhydride was added to the solution and the mixture was stirred at 70°C for 2 h. Toluene used as the solvent was distilled off from the mixture by heating under reduced pressure. 400 mℓ of water was added to the residue to obtain a solution. An aqueous solution of 92.4 g (0.733 mol) of sodium sulfite was added dropwise thereto at room temperature for about 1 h and then the mixture was stirred at 70°C for 1 h. Water was distilled off from the reaction mixture. About 1 ℓ of ethanol was added thereto and, after reflux for about 1 h, an insoluble white precipitate thus formed was recovered by filtration. It was dried in a desiccator under reduced pressure to obtain 343.9 g of a white powder (yield: 76.6% based on maleic anhydride).

The structure of the product was confirmed in the same manner as that of Example 1.

Example 3

200 g (0.568 mol) of decylglucoside having a degree of sugar condensation of 1.20 was heated to 130°C. 55.6 g (0.568 mol) of maleic anhydride melted by heating was added dropwise thereto under stirring for 30 min. After the completion of the addition, the mixture was stirred at 130°C for additional 30 min. 200 mℓ of water was added thereto to form an aqueous solution. The aqueous solution was cooled to 60°C. 500 mℓ of an aqueous solution of 66.7 g (0.529 mol) of sodium sulfite was added dropwise thereto under stirring for about 30 min and then the mixture was stirred at 80°C for 2 h. Water was distilled off from the reaction mixture. 800 mℓ of ethanol was added to the solid residue. An insoluble matter was recovered by filtration and dried under reduced pressure to obtain 261.7 g of a white powder (yield: 79.9% based on decylglucoside).

The structure of the product was confirmed from the mass spectrum by FAB ionization method.

Mass spectrum (FAB ionization method):

Molecular ion peaks (+H type and +Na type) corresponding to disodium salt of decylmonoglucoside half ester of monosulfosuccinic acid and disodium salt of decyldiglucoside half ester of monosulfosuccinic acid were detected:

disodium salt of decylmonoglucoside half ester of monosulfosuccinic acid:

$(C_{20}H_{34}O_{12}SNa_2 = 544)$: 545 $(M+H)_+$,

567 $(M+Na)^+$,

disodium salt of decyldiglucoside half ester of monosulfosuccinic acid:

$(C_{26}H_{44}O_{17}SNa_2 = 706)$: 707 $(M+H)^+$,

729 $(M+Na)^+$

## Claims

1. A glycoside ester of sulfosuccinic acid of the following general formula (I):

   A(Gn)[(R'O)xB]y (I)

   wherein:

   Gn represents a sugar residue remaining after removing hydrogen atoms from all of the non-glycosidic hydroxyl groups and glycosidic hydroxyl groups of a reducing sugar having 5 or 6 carbon atoms or a condensate thereof in which n represents the degree of condensation which has an average value from 1 to 10;

   A represents an $R^2(OR^3)_z$ group bound to the sugar residue Gn through an O-glycoside bond in which $R^2$ represents a straight chain or branched alkyl, alkenyl or alkylphenyl group having 6 to 22 carbon atoms, $R^3$ represents an alkylene group having 2 to 4 carbon atoms and z represents a number having an average value from 0 to 20;

   $R^1$ represents an alkylene group having 2 to 4 carbon atoms, one end of which forms an ether bond with an oxygen atom of a non-glycosidic hydroxyl group of the sugar residue Gn and the other end of which forms an ester bond with a sulfosuccinoyl group;

   x represents the ratio of the total molar number of alkylene oxide groups added to the non-glycosidic hydroxyl groups of the reducing sugar having 5 or 6 carbon atoms or a condensate thereof to y, and has a value from 0 to 10;

   y represents the number of non-glycosidic hydroxyl groups in the reducing sugar having 5 or 6 carbon atoms or a condensate thereof; and

   B is hydrogen or

   $$-CO-CH2CH(SO3M)-CO2M,$$

   provided that at least one B residue is is

   $$-CO-CH2CH(SO3M)-CO2M;$$

   and

   M represents residues that may be the same of different from each other and each is a hydrogen atom, an alkli metal, an alkaline earth metal, ammonium, mono-, di- or trialkanolammonium having 2 or 3 carbon atoms, an alkyl-substituted ammonium having 1 to 5 carbon atoms, or a basic amino acid group.

2. A glycoside ester of sulfosuccinic acid according to Claim 1 wherein the sugar residue Gn in the general formula (I) is that remaining after removal of the hydrogen atoms from all of the non-glycosidic hydroxyl groups and glycosidic hydroxyl groups of glucose or a condensate thereof.

3. A process for producing a glycoside ester of sulfosuccinic acid defined in Claim 1 by reacting a glycoside compound or an alkylene oxide adduct thereof of the following general formula (II) or a mixture of them with maleic anhydride to form a compound of the following general formula (III), and reacting this compound with an alkali metal sulfite and/or an acidic alkali metal sulfite containing an alkali metal hydroxide:

$$A(Gn)[(R'O)xH]y \qquad (II)$$

wherein:

Gn represents a sugar residue remaining after removing hydrogen atoms from all of the non-glycosidic hydroxyl groups and glycosidic hydroxyl groups of a reducing sugar having 5 or 6 carbon atoms or its condensate in which n represents the degree of condensation which has an average value from 1 to 10;

A represents an $R^2(OR^3)_z$ group bound to the sugar residue Gn through an O-glycoside bond in which $R^2$ represents a straight chain or branched alkyl, alkenyl or alkylphenyl group having 6 to 22 carbon atoms, $R^3$ represents an alkylene group having 2 to 4 carbon atoms and z represents a number having an average value from 0 to 20;

$R^1$ represents an alkylene group having 2 to 4 carbon atoms, one end of which forms an ether bond with an oxygen atom of a non-glycosidic hydroxyl group of the sugar residue Gn and the other end of which is bound to a hydroxyl group;

x represents the ratio of the total molar number of alkylene oxide groups added to the non-glycosidic hydroxyl groups of the reducing sugar having 5 or 6 carbon atoms or a condensate thereof to y, and has a value from 0 to 10; and

y represents the number of the non-glycosidic hydroxyl groups in the reducing sugar having 5 or 6 carbon atoms or a condensate thereof, and

$$A(Gn)[(R'O)xE]y \qquad (III)$$

wherein:

A, Gn, x and y are as defined above;

$R^1$ represents an alkylene group having 2 to 4 carbon atoms, one end of which forms an ether bonded with an oxygen atom of a non-glycosidic hydroxyl group of the sugar residue Gn and the other end of which is bound to the ester group;

E is hydrogen or -CO-CH=CH-SO3M, provided that at least one of the E residues is -CO-CH=CH-SO3M; and the M residues may be the same or different from each other and each represents a hydrogen atom, a alkali metal, an alkaline earth metal, ammonium, mono-, di- or trialkanolammonium having 2 or 3 carbon atoms, an alkyl-substituted ammonium having 1 to 5 carbon atoms, or a basic amino acid group.